# EUROPEAN PATENT SPECIFICATION

(11) **EP 3 528 708 B1**
(45) Date of publication and mention of the grant of the patent: **29.06.2022**
(21) Application number: 17818886.8
(22) Date of filing: 11.04.2017
(51) Int. Cl.: A61B 8/00, G01N 29/24, G01S 7/52, G10K 11/00, H01R 13/00, H01R 13/62, H01R 13/70, A61B 1/00, A61B 17/00, G01S 15/89

(54) **APPARATUS AND METHOD FOR SEMI-AUTOMATIC ULTRASOUND TRANSDUCER CONNECTOR**
VORRICHTUNG UND VERFAHREN FÜR HALBAUTOMATISCHEN ULTRASCHALLWANDLERVERBINDER
APPAREIL ET PROCÉDÉ DE CONNECTEUR DE TRANSDUCTEUR À ULTRASONS SEMI-AUTOMATIQUE

(30) Priority: 30.06.2016 US 201615199019
(43) Date of publication of application: 28.08.2019
(73) Proprietor: Edan Instruments, Inc., Shenzhen, Guangdong 518122 (CN)
(72) Inventor: HENDERSON, Richard, Shenzhen Guangdong 518122 (CN); MURPHY, Sean, Shenzhen Guangdong 518122 (CN)
(74) Representative: dompatent von Kreisler Selting Werner - Partnerschaft von Patent- und Rechtsanwälten mbB
(86) International application number: PCT/CN2017/080085
(87) International publication number: WO 2018/000889

(56) References cited:
- CN-A- 1 672 639
- CN-A- 104 205 206
- CN-A- 104 323 795
- KR-A- 20140 100 213
- US-A- 5 882 310
- US-A1- 2008 114 249
- US-A1- 2008 194 964
- US-A1- 2013 165 790
- US-A1- 2015 025 390
- US-A1- 2017 000 456
- US-B1- 6 375 619
- US-B1- 6 440 076

## Description

### CROSS-REFERENCE TO RELATED APPLICATIONS

### BACKGROUND

The present inventive concept relates to ultrasound transducer connectors and, more specifically, to an apparatus and a method that semi-automatically locks an ultrasound transducer connector to a terminal of an ultrasound system.

An ultrasound system images a patient by producing an emitted ultrasonic wave with a transducer. The transducer measures return echoes of these waves to provide data and images regarding the patient. Typically, an ultrasound system consists of a transducer, a processor that processes echoes received by the transducer, a display device that displays data and ultrasound images received and a user interface that allows the user to operate the ultrasound device. The ultrasound transducer has on one end a probe, and on the other end an ultrasound transducer connector that can be plugged into a terminal of an ultrasound system. The ultrasound transducer and the ultrasound system are thus connected via the ultrasound transducer connector. D1 (US2013165790A1) provides an ultrasound diagnostic apparatus and a method of controlling the same. D2 (US6375619B1) discloses an ultrasonic diagnostic apparatus comprising an apparatus main body and an ultrasonic scope detachably attached to the main body. D3 (US2015025390A1) discloses a reversible transducer connector allowing the connector to be inserted within the receptacle in more than one orientation. D4 (US5882310A) discloses a plurality of ultrasound imaging system receptacles which are arranged either vertically one above the other or horizontally side-by-side, each receptacle having an insertion slot for receiving the contact pads of an inserted ultrasound transducer connector. D5 (US2008114249A1) discloses a method, system, and device for conducting ultrasound interrogation of a medium. D6 (KR20140100213A) relates to a probe table and an ultrasound imaging apparatus, in which the probe table includes an ultrasound probe connection part for connecting a plurality of ultrasonic probes and a display part which displays the operation condition or the connection condition of at least one ultrasonic probe which is connected to the ultrasound probe connection part.

### SUMMARY

One embodiment relates to an apparatus for locking a transducer connector to a transducer interface of an ultrasound system. The apparatus includes a sensor configured to acquire data regarding a position of the transducer connector relative to the transducer interface, a locking system configured to facilitate locking of the transducer connector to the transducer interface, and a processing circuit communicably coupled to the sensor and the locking system. The processing circuit is configured to interpret the data acquire by the sensor and provide a lock command to the locking system to lock the transducer connector to the transducer interface based on the data indicating the transducer connector is engaged with the transducer interface.

Another embodiment relates to a method for locking a transducer connector to a transducer interface of an ultrasound system. The method includes acquiring, by a sensor, data regarding a position of the transducer connector relative to the transducer interface; interpreting, by a processing circuit, the data; and providing, by the processing circuit, a lock command to a locking system to lock the transducer connector to the transducer interface based on the data indicating the transducer connector is engaged with the transducer interface.

Still another embodiment relates to an apparatus for locking an ultrasound transducer connector to a transducer interface of an ultrasound system. The apparatus includes a locking system configured to facilitate automatic locking of the ultrasound transducer connector to the transducer interface responsive to the ultrasound transducer connector interfacing with the transducer interface, and a processing circuit communicably coupled the locking system and configured to provide a lock command to the locking system to lock the ultrasound transducer connector to the transducer interface responsive to the ultrasound transducer connector interfacing with the transducer interface.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

FIG. 1 illustrates a perspective view of an ultrasound system, according to one embodiment.
FIG. 2 illustrates a front view of an ultrasound system, according to one embodiment.
FIG. 3 illustrates a block diagram of components of an ultrasound system, according to one embodiment.
FIG. 4 is a schematic block diagram of an ultrasound transducer connector lock apparatus, according to one embodiment.
FIG. 5 is a perspective view of an ultrasound transducer connector lock apparatus, according to one embodiment.
FIG. 6 is a perspective view of an ultrasound transducer connector lock apparatus employing a gear arrangement, according to one embodiment.
FIG. 7 is a perspective view of an ultrasound transducer connector lock apparatus employing a direct connection arrangement, according to one embodiment.
FIG. 8 is a perspective view of an ultrasound transducer connector lock apparatus employing a linkage mechanism arrangement, according to one embodiment.
FIGS. 9A-9B illustrate various release mechanisms, according to one embodiment.
FIG. 10 is a flow diagram of a method for locking an ultrasound transducer to an ultrasound system, according to one embodiment.
FIG. 11 is a flow diagram of a method for unlocking an ultrasound transducer from an ultrasound system, according to one embodiment.

### DETAILED DESCRIPTION

In the following detailed description, reference is made to the accompanying drawings, drawings, which form a part thereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented here.

Referring to the Figures generally, a semi-automatic locking mechanism for a transducer connector facilitates automatic locking of the transducer connector (e.g., an ultrasound transducer connector, etc.) to a transducer interface of an ultrasound system. The locking device also facilitates the disengagement of the transducer connector from the transducer interface responsive to a release command. Traditionally, a user manually activates a lever to latch and unlatch an ultrasound transducer connector to and from a transducer interface of an ultrasound system. Typically, the levers are either rotational or a "flip paddle" and require a force that may aggravate the user's hand to lock the ultrasound transducer connector to the ultrasound system. Over time, the latch and unlatch operation may become tedious, annoying, and uncomfortable to perform routinely. Additionally, the manual nature of the connection may allow for human error which results in damage to the ultrasound transducer connector and/or to the ultrasound system (e.g., attempting to force a connection between incompatible components, applying excessive force, and/or otherwise causing damage to components) and/or causes the system to not function as intended (e.g., an incomplete connection between components from a user not fully actuating a lever or other device).

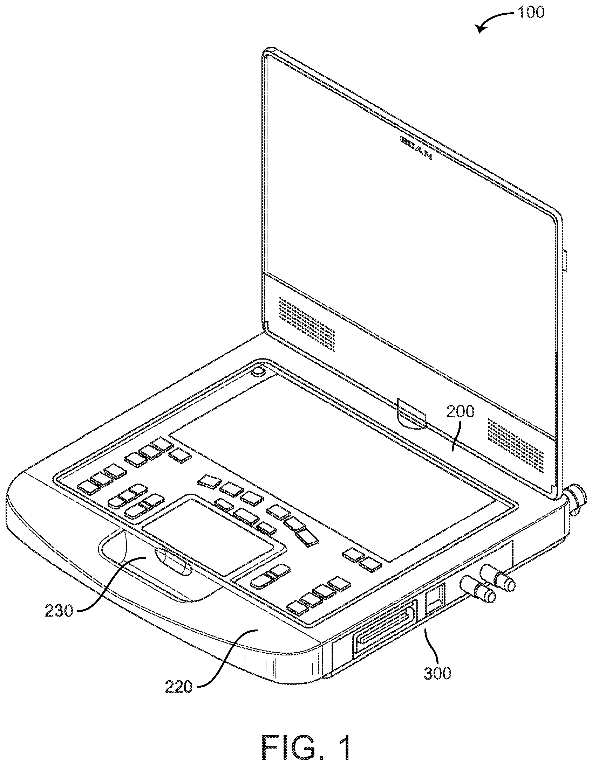

Referring to FIG. 1, one embodiment of ultrasound system 100 is illustrated. As shown in FIG. 1, ultrasound system 100 is structured as a portable ultrasound system. In one embodiment, ultrasound system 100 is a laptop. In other embodiments, ultrasound system 100 is another type of portable device (e.g., a tablet, a smartphone, a form factor device, etc.). In a comparative embodiment, ultrasound system 100 is structured as a stationary ultrasound device.

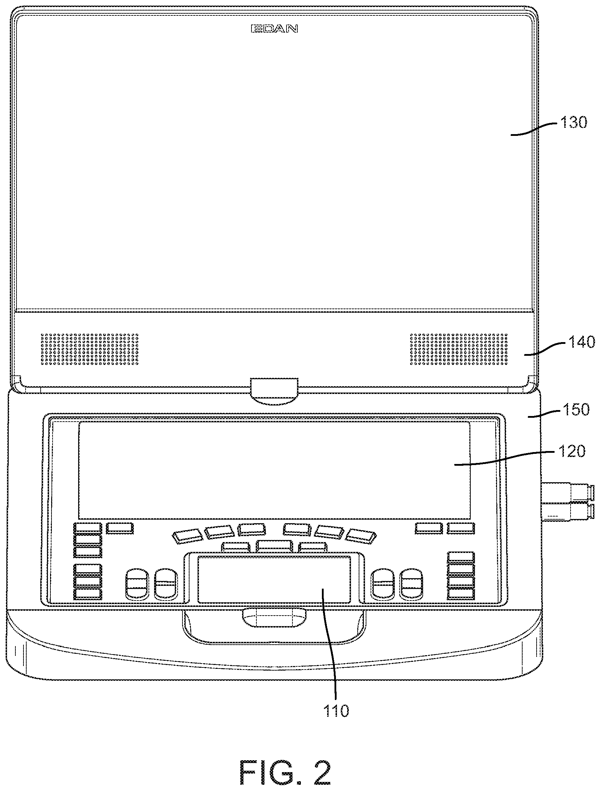

Referring to FIG. 2, a front view of one embodiment of ultrasound system 100 is illustrated. Main housing 150 houses components of ultrasound system 100. In some embodiments, the components housed within main housing 150 include ergonomic handle system 220, status indicator system 230, and locking system 300 as shown in FIG. 1. Main housing 150 may also be configured to support electronics modules which may be replaced and/or upgraded due to the modular construction of ultrasound system 100. In some embodiments, ultrasound system 100 includes display housing 140. Display housing 140 may include display support system 200 as shown in FIG. 1. In some embodiments, ultrasound system 100 includes touchpad or touchscreen 110 for receiving user inputs and displaying information, touchscreen 120 for receiving user inputs and displaying information, and main screen 130 for displaying information.

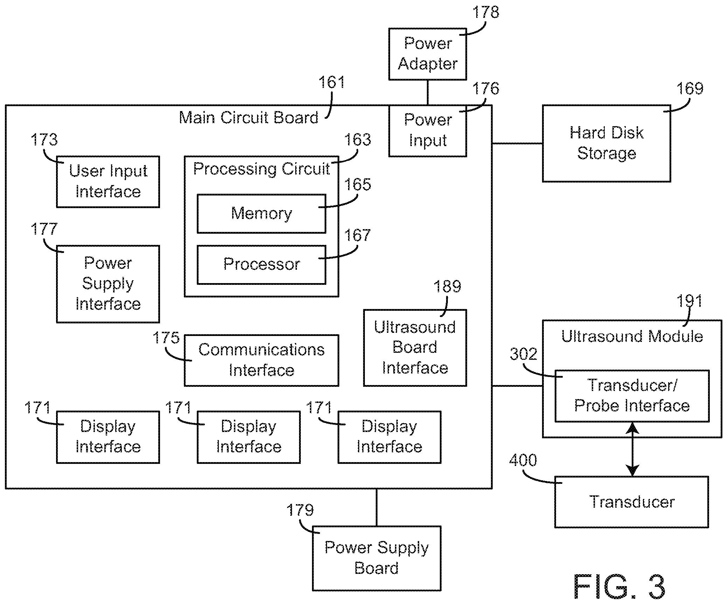

Referring to FIG. 3, a block diagram shows internal components of one embodiment of ultrasound system 100. Ultrasound system 100 includes main circuit board 161. Main circuit board 161 carries out computing tasks to support the functions of ultrasound system 100 and provides connection and communication between various components of ultrasound system 100. In some embodiments, main circuit board 161 is configured so as to be a replaceable and/or upgradable module.

To perform computational, control, and/or communication tasks, main circuit board 161 includes processing circuit 163. Processing circuit 163 is configured to perform general processing and to perform processing and computational tasks associated with specific functions of ultrasound system 100. For example, processing circuit 163 may perform calculations and/or operations related to producing an image from signals and or data provided by ultrasound equipment, running an operating system for ultrasound system 100, receiving user inputs, etc. Processing circuit 163 may include memory 165 and processor 167 for use in processing tasks. For example, processing circuit may perform calculations and/or operations.

Processor 167 may be, or may include, one or more microprocessors, application specific integrated circuits (ASICs), circuits containing one or more processing components, a group of distributed processing components, circuitry for supporting a microprocessor, or other hardware configured for processing. Processor 167 is configured to execute computer code. The The computer code may be stored in memory 165 to complete and facilitate the activities described herein with respect to ultrasound system 100. In other embodiments, the computer code may be retrieved and provided to processor 167 from hard disk storage 169 or communications interface 175 (e.g., the computer code may be provided from a source external to main circuit board 161).

Memory 165 can be any volatile or non-volatile computer-readable storage medium capable of storing data or computer code relating to the activities described herein. For example, memory 165 may include modules which are computer code modules (e.g., executable code, object code, source code, script code, machine code, etc.) configured for execution by processor 167. Memory 165 may include computer executable code related to functions including ultrasound imaging, battery management, handling user inputs, displaying data, transmitting and receiving data using a wireless communication device, etc. In some embodiments, processing circuit 163 may represent a collection of multiple processing devices (e.g., multiple processors, etc.). In such cases, processor 167 represents the collective processors of the devices and memory 165 represents the collective storage devices of the devices. When executed by processor 167, processing circuit 163 is configured to complete the activities described herein as associated with ultrasound system 100.

Hard disk storage 169 may be a part of memory 165 and/or used for non-volatile long term storage in ultrasound system 100. Hard disk storage 169 may store local files, temporary files, ultrasound images, patient data, an operating system, executable code, and any other data for supporting the activities of ultrasound system 100 described herein. In some embodiments, hard disk storage is embedded on main circuit board 161. In other embodiments, hard disk storage 169 is located remote from main circuit board 161 and coupled thereto to allow for the transfer of data, electrical power, and/or control signals. Hard disk 169 may be an optical drive, magnetic drive, a solid state hard drive, flash memory, etc.

In some embodiments, main circuit board 161 includes communications interface 175. 175. Communications interface 175 may include connections which enable communication between components of main circuit board 161 and communications hardware. For example, communications interface 175 may provide a connection between main circuit board 161 and a network device (e.g., a network card, a wireless transmitter/receiver, etc.). In further embodiments, communications interface 175 may include additional circuitry to support the functionality of attached communications hardware or to facilitate the transfer of data between communications hardware and main circuit board 161. In other embodiments, communications interface 175 may be a system on a chip (SOC) or other integrated system which allows for transmission of data and reception of data. In such a case, communications interface 175 may be be coupled directly to main circuit board 161 as either a removable package or embedded package.

Some embodiments of ultrasound system 100 include power supply board 179. Power Power supply board 179 includes components and circuitry for delivering power to components and devices within and/or attached to ultrasound system 100. In some embodiments, power supply board 179 includes components for alternating current and direct current conversion, for transforming voltage, for delivering a steady power supply, etc. These components may include include transformers, capacitors, modulators, etc. to perform the above functions. In further embodiments, power supply board 179 includes circuitry for determining the available power of a battery power source. In other embodiments, power supply board 179 includes circuitry for switching between power sources. For example, power supply board 179 may draw power from from a backup battery while a main battery is switched. In further embodiments, power supply board 179 includes circuitry to operate as an uninterruptable power supply in conjunction with a backup battery. Power supply board 179 also includes a connection to main circuit board 161. This connection may allow power supply board 179 to send and receive information from main circuit board 161. For example, power supply board 179 may send information to main circuit board 161 allowing for the determination of remaining battery power. The connection to main circuit board 161 may also allow main circuit board 161 to send commands to power supply board 179. For example, main circuit board 161 may send a command to power supply board 179 to switch from one source of power to another (e.g., to switch to a backup battery while a main battery is switched). In some embodiments, power supply board 179 is configured to be a module. In such cases, power supply board 179 may be configured so as to be a replaceable and/or upgradable module.

Main circuit board 161 may also include power supply interface 177 which facilitates the above described communication between power supply board 179 and main circuit board 161. Power supply interface 177 may include connections which enable communication between components of main circuit board 161 and power supply board 179. In further embodiments, power supply interface 177 includes additional circuitry to support the functionality of power supply board 179. For example, power supply interface 177 may include circuitry to facilitate the calculation of remaining battery power, manage switching between available power sources, etc. In other embodiments, the above described functions of power supply board 179 may be carried out by power supply interface 177. For example, power supply interface 177 may be a SOC or other integrated system. In such a case, power supply interface 177 may be coupled directly to main circuit board 161 as either a removable package or embedded package.

With continued reference to FIG. 3, some embodiments of main circuit board 161 include user input interface 173. User input interface 173 may include connections which enable communication between components of main circuit board 161 and user input device hardware. For example, user input interface 173 may provide a connection between main circuit board 161 and a capacitive touchscreen, resistive touchscreen, mouse, keyboard, buttons, and/or a controller for the proceeding. In one embodiment, user input interface 173 couples controllers for touchpad or touchscreen 110, touchscreen 1 20, and main screen 130 to main circuit board 161. In other embodiments, user input interface 173 includes controller circuitry for touchpad or touchscreen 110, touchscreen 120, and main screen 130. In some embodiments, main circuit board 161 includes a plurality of user input interfaces 173. For example, each user input interface 173 may be associated with a single input device (e.g., touchpad or touchscreen 110, touchscreen 120, a keyboard, buttons, etc.).

In further embodiments, user input interface 173 may include additional circuitry to support the functionality of attached user input hardware or to facilitate the transfer of data between user input hardware and main circuit board 161. For example, user input interface 173 may include controller circuitry so as to function as a touchscreen controller. User input interface 173 may also include circuitry for controlling haptic feedback devices associated with user input hardware. In other embodiments, user input interface 173 may be a SOC or other integrated system which allows for receiving user inputs or otherwise controlling user input hardware. In such a case, user input interface 173 may be coupled directly to main circuit board board 161 as either a removable package or embedded package.

Main circuit board 161 may also include ultrasound board interface 189 which facilitates communication between ultrasound board 191 and main circuit board 161. Ultrasound board interface 189 may include connections which enable communication between components of main circuit board 161 and ultrasound board 191. In further embodiments, ultrasound board interface 189 includes additional circuitry to support the functionality of ultrasound board 191. For example, ultrasound board interface 189 may include circuitry to facilitate the calculation of parameters used in generating an image from ultrasound data provided by ultrasound board 191. In some embodiments, ultrasound board interface 189 is a SOC or other integrated system. In such a case, ultrasound board interface 189 may be coupled directly to main circuit board 161 as either a removable package or embedded package.

In other embodiments, ultrasound board interface 189 includes connections which facilitate use of a modular ultrasound board 191. Ultrasound board 191 may be a module (e.g., ultrasound module) capable of performing functions related to ultrasound imaging (e.g., multiplexing sensor signals from an ultrasound probe/transducer, controlling the frequency of ultrasonic waves produced by an ultrasound probe/transducer, etc.). The connections of ultrasound board interface 189 may facilitate replacement of ultrasound board 191 (e.g., to replace ultrasound board 191 with an upgraded board or a board for a different application). For example, ultrasound board interface 189 may include connections which assist in accurately aligning ultrasound board 191 and/or reducing the likelihood of damage to ultrasound board 191 during removal and or attachment (e.g., by reducing the force required to connect and/or remove the board, by assisting, with a mechanical advantage, the connection and/or removal of the board, etc.).

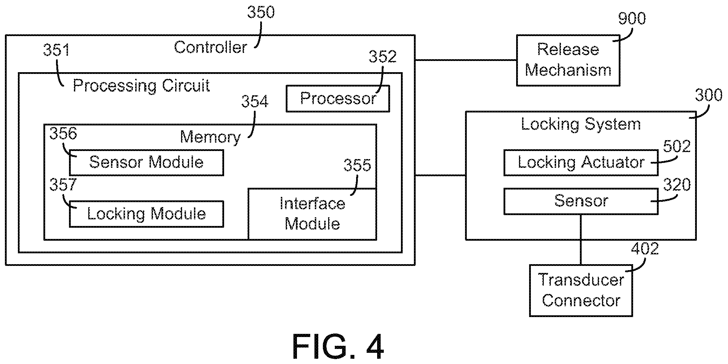

In embodiments of ultrasound device 100 including ultrasound module 191, ultrasound module 191 includes components and circuitry for supporting ultrasound imaging functions of ultrasound device 100. In some embodiments, ultrasound module 191 includes integrated circuits, processors, and memory. Ultrasound module 191 may also include one or more transducer/probe interfaces 302. Transducer/probe interface 302 enables a connector (e.g., transducer connector 402 of FIGS. 4-8, etc.) of an ultrasound transducer/probe (e.g., a probe with a socket type connector, a probe with a pin type connector, etc.), shown as transducer 400, to interface with ultrasound module 191. For example, transducer/probe interface 302 may include circuitry and/or hardware connecting transducer 400 to ultrasound module 191 for the transfer of electrical power and/or data. Transducer/probe interface 302 may include hardware which locks transducer 400 into place (e.g., a slot which accepts a pin on the ultrasound transducer/probe when the ultrasound transducer/probe is rotated, a locking lever system, etc.). In some embodiments, ultrasound module 191 includes two or more transducer/probe interfaces 302 to allow the connection of a plurality of transducers 400.

With continued reference to FIG. 3, some embodiments of main circuit board 161 include display interface 171. Display interface 171 may include connections which enable communication between components of main circuit board 161 and display device hardware. For example, display interface 171 may provide a connection between main circuit board 161 and a liquid crystal display, a plasma display, a cathode ray tube display, a light emitting diode display, and/or a display controller or graphics processing unit for the proceeding or other types of display hardware. In some embodiments, the connection of display hardware to main circuit board 161 by display interface 171 allows a processor or dedicated graphics processing unit on main circuit board 161 to control and/or send data to display hardware. Display interface 171 may be configured to send display data to display device hardware in order to produce an image. In some embodiments, main circuit board 161 includes multiple display interfaces 171 for multiple display devices (e.g., three display interfaces 171 connect three displays to main circuit board 161). In other embodiments, one display interface 171 may connect and/or support multiple displays. In one embodiment, three display interfaces 171 couple touchpad or touchscreen 110, touchscreen 120, and main screen 130 to main circuit board 161.

In further embodiments, display interface 171 may include additional circuitry to support the functionality of attached display hardware or to facilitate the transfer of data between display hardware and main circuit board 161. For example, display interface 171 may include controller circuitry, a graphics processing unit, video display controller, etc. In some embodiments, display interface 171 may be a SOC or other integrated system which allows for displaying images with display hardware or otherwise controlling display hardware. Display interface 171 may be coupled directly to main circuit board 161 as either a removable package or embedded package. Processing circuit 163 in conjunction with one or more display interfaces 171 may display images on one or more of touchpad or touchscreen 110, touchscreen, 120, and main screen 130.

Generally, a logic circuit or logic circuitry handles user inputs through the user interface of ultrasound system 100. The logic circuit processes user inputs and responds to user inputs. This may include controlling hardware components such as displays, networking devices, ultrasound transducers, etc. Additionally, the logic circuit may respond to user inputs by taking an action through a software component of ultrasound system 100. For example, the logic circuit may alter the priority of a hardware device for purposes of allocating physical resources such as processing resourses, memory, input devices, output devices, etc.

With reference to FIG. 3, the logic circuit controls ultrasound system 100. The logic logic circuit may control ultrasound system 100 through a combination of programming, default states, user inputs, event response, outputs, etc. In some embodiments, the logic circuit is incorporated into main circuit board 161. For example, the logic circuit may be implemented as processing circuit 163. Alternatively, processing circuit 163 may perform the functions of the logic circuit set above including acquiring user inputs, processing user inputs, and controlling hardware. In some embodiments, processing circuit 163 makes use of other resources included within main board 161 in controlling ultrasound system 100. For example, processing circuit 163 may receive user inputs through user input interface 173 and control hardware through display interface 171, communications interface 175, ultrasound board interface 189, ultrasound board 191, and/or user input interface 173. In some embodiments, user inputs and display outputs occur through an operating system and/or graphical user interface (GUI). Computer code and/or instructions for implementing the operating system and/or GUI through event handling, input handling, hardware control, etc. may be stored in memory 165. In further embodiments, computer code and/or instructions regarding the above may be stored in hard disk storage 169 and/or acquired or received by processing circuit 163 using communications interface 175 and a communications device. The operating system and/or GUI may be implemented across one or more of main screen 130, touchscreen 120, and touchpad or touchscreen 110 shown in FIG. 2.

Referring now to FIG. 4, the function and structure of controller 350 are shown according to one embodiment. Controller 350 is shown to include processing circuit 351 including processor 352 and memory 354. Processor 352 may be implemented as a generalpurpose processor, an application specific integrated circuit (ASIC), one or more field programmable gate arrays (FPGAs), a digital signal processor (DSP), a group of processing components, or other suitable electronic processing components. One or more memory devices 354 (e.g., RAM, ROM, Flash Memory, hard disk storage, etc.) may store data and/or computer code for facilitating the various processes described herein. Thus, one or more memory devices 354 may be communicably connected to processor 352 and provide computer code or instructions to processor 352 for executing the processes described in regard to controller 350 herein. Moreover, one or more memory devices 354 may be or include tangible, non-transient volatile memory or non-volatile memory. In some embodiments, one or more memory devices 354 may include database components, object code components, script components, or any other type of information structure for supporting the various activities and information structures described herein.

Memory 354 is shown to include various modules for completing processes described described herein. More particularly, memory 354 includes modules configured to semi-automatically couple transducer connector 402 of a transducer (e.g., transducer 400 shown in FIG. 3) to locking system 300. While various modules with particular functionality are shown in in FIG. 4, it will be understood that controller 350 and memory 354 may include any number of modules for completing the functions described herein. For example, the activities of multiple modules may be combined as a single module and additional modules with additional functionality may be included. Further, it will be understood that controller 350 may further control other processes beyond the scope of the present disclosure.

Communication between and among the components of controller 350, locking system 300, and transducer 400 may be via any number of wired or wireless connections. For example, a wired connection may include a serial cable, a fiber optic cable, a CAT5 cable, or any other form of wired connection. In comparison, a wireless connection may include the Internet, Wi-Fi, cellular, radio, Bluetooth, etc. In one embodiment, a controller area network (CAN) bus provides the exchange of signals, information, and/or data. The CAN bus includes any number of wired and wireless connections. Because controller 350 is communicably coupled to the systems and components of ultrasound system 100 of FIGS. 1-3, controller 350 may receive data from one or more of the components shown in FIGS. 1-3. For example, the data may include imaging data acquired via one or more transducers 400. As another example, the data may include an input from a sensor 320 and/or a release mechanism 900. As described more fully herein, controller 350 may automatically lock to locking system 300 responsive to the data or unlock transducer connector 402 responsive to the input.

As shown in FIG. 4, controller 350 includes an interface module 355, a sensor module module 356, and a locking module 357. The interface module 355 may be communicably coupled to a variety of components, including locking system 300 and release mechanism 900. Locking system 300 includes sensor 320 and actuator 502. Sensor 320 is configured to automatically detect when transducer connector 402 of transducer 400 is in position (e.g., interfacing with transducer interface 302 shown in FIG. 3, via any suitable wireless communication protocol such as Bluetooth, RFID, infrared, radio frequency, etc.) such that actuator 502 may be engaged, securing transducer connector 402 to ultrasound system 100. Interface module 355 is communicably coupled to sensor module 356 such that the data may be communicated to sensor module 356. Interface module 355 is further configured to receive a release command (e.g., an unlock command, a disengagement command, etc.) from release mechanism 900. The release command is configured to provide a signal to controller 350 350 that an operator desires to release (e.g., decouple, disengage, unlock, etc.) transducer connector 402 from locking system 300 (e.g., after use, to switch transducers 400, etc.) . Interface module 355 is communicably coupled to locking module 357 such that the release command may be communicated to locking module 357.

Sensor module 356 is configured to receive and interpret the data from interface module 355 regarding transducer connector 402. Sensor module 356 may verify that the respective transducer connector 402 attempting to be coupled to locking system 300 of ultrasound system 100 is appropriate (e.g., transducer connector 402 is compatible with locking system 300, both mechanically and software compatible, etc.) and/or transducer connector 402 is properly interfaced with transducer interface 302 (e.g., aligned properly, fully inserted/engaged, etc.). For example, transducer connector 402 may include an identification tag (e.g., an RFID tag, etc.), or another type of identification method may be used, such that sensor module 356 may determine whether transducer connector 402 is compatible with locking system 300. The verification may substantially prevent any excessive damage to transducer connector 402 and/or locking system 300. In another example, transducer connector 402 may only be partially inserted into transducer interface 302 such that locking mechanism is unable to lock transducer connector 402 to ultrasound system 100.

In some embodiments, locking system 300 may include an indicator lamp/light (e.g., an an LED, etc.) that is configured to flash a designated color (e.g., red, etc.) if sensor module 356 determines that transducer connector 402 is not compatible with ultrasound system 100 / locking system 300 and/or transducer connector 402 is not properly interfaced with transducer interface 302. In other embodiments, locking system 300 may include an indicator lamp/light (e.g., an LED, etc.) that is configured to flash a different designated color (e.g., green, etc.) if sensor module 356 determines that transducer connector 402 is compatible with ultrasound system 100 / locking system 300 and/or transducer connector 402 is properly interfaced with transducer interface 302. In a comparative embodiment, the indication regarding the compatibility or engagement of transducer connector 402 with ultrasound system 100 is or includes a different type of indication (e.g., a visual message/notification provided by a display of ultrasound system 100, an audible indication provided by a speaker of ultrasound system 100, haptic feedback provided by a vibratory element of ultrasound system 100, etc.). In some embodiments, a combination of indications may be provided to indicate compatibility, incompatibility, that the transducer connector is or is not properly interfaced with transducer interface 302, and/or other information. For example, if transducer connector 402 is incompatible, an indicator may be illuminated (e.g., red) and an audio tone (e.g., an alarm tone) may be played by a speaker included in locking system 300 or elsewhere in ultrasound system 100. As an additional example, if transducer connector 402 is compatible and/or is properly interfaced with transducer interface 302, an indicator may be illuminated (e.g., yellow) and an audio tone (e.g., a chime) may be played. Further indicator type combinations are used in various embodiments.

In some embodiments, the indicator is located on transducer connector 402 or elsewhere on the transducer assembly (e.g., on transducer 400). The indicator may receive an input (e.g., signal, instruction, command, etc.) from controller 350 via interface module 355 and/or locking system 300 which causes the indicator to provide an indication (e.g., according to a determination based on input from sensor 320 that transducer connector 402 is or is not compatible and/or transducer connector 402 is or is not properly interfaced with transducer interface 302). In some embodiments, the indicator is located on the transducer assembly (e.g., connector 402, transducer 400, etc.) and ultrasound system 100 does not include an indicator. In one comparative embodiment, the indicator is located on ultrasound system 100 and the transducer assembly does not include an indicator. In still another comparative embodiment, both ultrasound system 100 and the transducer assembly include an indicator.

Locking module 357 is configured to send commands (e.g., a locking command, etc.) to actuator 502 to facilitate automatic locking of transducer connector 402 to transducer interface 302 responsive to sensor 320 sensing the presence of transducer connector 402 (e.g., upon determination by sensor module 356 that transducer connector 402 is compatible and/or fully engaged, etc.). Transducer connector 402 is thereby releasably secured to ultrasound device such that imaging data may be transmitted from transducer 400 to ultrasound module 191 of ultrasound system 100. Locking module 357 is further configured to receive an input from release mechanism 900 via interface module 355. Locking module 357 is configured to send a release command to actuator 502 responsive to the input to facilitate the selective release (e.g., disengagement, unlocking, etc.) of transducer connector 402 from transducer interface 302.

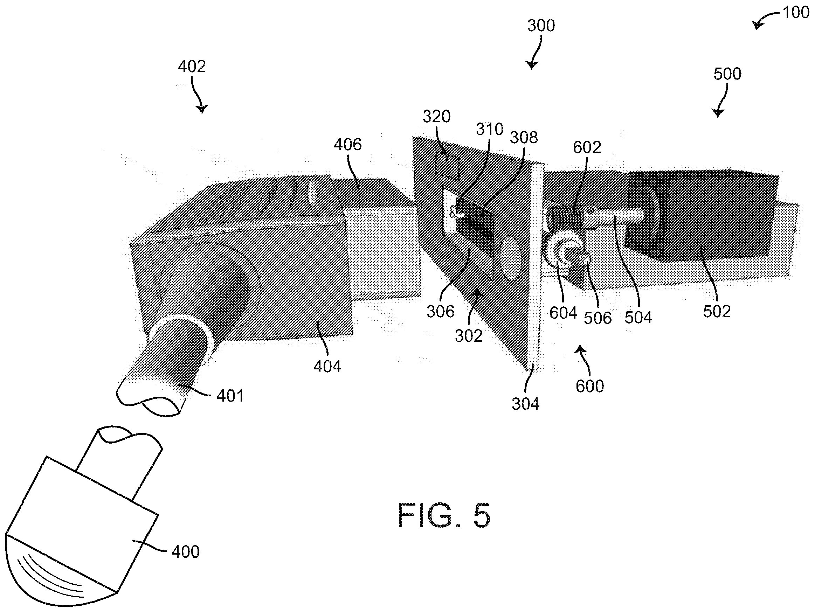

According to the exemplary embodiments shown in FIGS. 5-8, locking system 300 facilitates the locking and unlocking of transducer 400 to ultrasound system 100 via the interaction between transducer connector 402 and transducer interface 302. As shown in FIGS. 5-8, transducer connector 402 includes a housing, shown as connector housing 404. Connector housing 404 is structured to receive a cable, shown as cable 401. As shown in FIG. 5, cable 401 couples transducer 400 to transducer connector 402. Cable 401 facilitates the transfer of imaging data from transducer 400 through transducer connector 402 to ultrasound system 100. In a comparative embodiment, transducer 400 wirelessly connects and transmits data through transducer connector 402 (e.g., via Bluetooth, Wi-Fi, radio frequency, etc.) to ultrasound system 100.

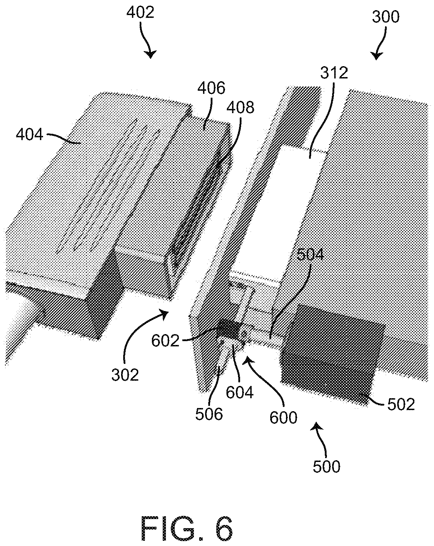

As shown in FIGS. 5-8, connector housing 404 includes an engagement feature, shown as male engagement feature 406. According to the exemplary embodiment, male engagement feature 406 is rectangular. In other embodiments, male engagement feature 406 is another shape (e.g., square, ovular, circular, triangular, etc.). In a comparative embodiment, the engagement feature is structured as a female engagement feature. As shown in FIGS. 6-8, male engagement feature 406 includes adapter 408. Adapter 408 is configured to transmit the imaging data from a transducer (e.g., transducer 400 shown in FIG. 5) to ultrasound module 191 of ultrasound device 100. In some embodiments, adapter 408 is a pin type adapter. In some embodiments, adapter 408 is a socket type adapter. In other embodiments, adapter 408 is another type of adapter.

As shown in FIGS. 5-8, locking system 300 includes transducer interface 302 and lock engagement mechanism 500. As shown in FIGS. 6-8, transducer interface 302 includes an engagement feature, shown as female engagement feature 312. As shown in FIG. 5, female engagement feature 312 is positioned within an aperture, shown as aperture 306, defined by housing wall 304 of ultrasound system 100. Female engagement feature 312 includes an adapter, shown as adapter 308. In one embodiment, adapter 308 is communicably coupled to ultrasound module 191 of ultrasound device 100. Female engagement feature 312 is structured to receive male engagement feature 406 such that adapter 308 of transducer interface 302 engages with adapter 408 of transducer connector 402, thereby facilitating the transfer of the imaging data from transducer 400 to ultrasound device 100. According to the exemplary embodiment, aperture 306 and female engagement feature 312 are rectangular such that each corresponds with male engagement feature 406. In other embodiments, aperture 306 and female engagement feature 312 are another shape (e.g., square, ovular, circular, triangular, etc.) to correspond with a different shaped male engagement feature 406. In some embodiments, female engagement feature 312 is structured to be a universal engagement feature such that female engagement feature 312 may receive a plurality of different shaped male engagement features 406. In this case, aperture 306 may have a shape that does not specifically correspond with the shape of a single male engagement feature 406, but corresponds with a plurality of different shaped male engagement features 406. In a comparative embodiment, the engagement feature of locking system 300 is structured as a male engagement feature configured to engage with a female transducer connector 402.

According to an exemplary embodiment, sensor 320 is configured to automatically detect when male engagement feature 406 of transducer connector 402 is engaged with female engagement feature 312 of locking system 300. Sensor 320 may be any device capable of detecting transducer connector 402. In some embodiments, sensor 320 is or includes a proximity sensor that is able to detect the presence of transducer connector 402 (e.g., without any physical contact, etc.). For example, a proximity sensor may emit an electromagnetic field or a beam of electromagnetic radiation (e.g., infrared, etc.), and look for changes in the field or a return signal. In other embodiments, sensor 320 is or includes a touch sensor that detects physical contact from transducer connector 402. Sensor 320 may be disposed along housing wall 304, positioned within female engagement feature 312, or any other suitable location on or within ultrasound system 100. Sensor 320 is configured to notify controller 350 when male engagement feature 406 of transducer connector 402 is properly engaged (e.g., fully inserted, etc.) with female engagement feature 312 of locking system 300. For example, proper engagement may include adapter 308 and adapter 408 interfacing, male engagement feature 406 being fully inserted into female engagement feature 312, ultrasound module 191 of ultrasound system 100 being compatible with transducer 400 (e.g., software compatibility, etc.), and/or other engagement characteristics. When proper engagement is achieved, controller 350 provides a locking command to lock engagement mechanism 500 to secure transducer connector 402 to transducer interface 302.

Referring still to FIGS. 5-8, lock engagement mechanism 500 includes an actuator device, shown as actuator 502, with an output, shown as output shaft 504. In one embodiment, actuator 502 is a rotational actuator such as a rotational motor. In another embodiment, actuator 502 is a linear actuator such as a solenoid or linear motor. In other embodiments, actuator 502 is another type of actuator device. Actuator 502 is configured to engage/disengage lock engagement mechanism 500 responsive to receiving a lock command or a release command from a controller (e.g., controller 350 shown in FIG. 4). Output shaft 504 is configured to transfer an output (e.g., a rotational output, a linear output, etc.) from actuator 502 to a connector shaft, shown as connector shaft 506. The output of actuator 502 drives connector shaft 506 to either engage or disengage a hook, shown as engagement hook 310. Lock engagement mechanism 500 may be configured to facilitate rotational movement of engagement hook 310 about an axis (e.g., a vertical axis, a longitudinal axis, a lateral axis, etc.) or linear movement in a designed direction (e.g., a vertical direction, a longitudinal direction, a lateral direction, etc.). Engagement hook 310 moves accordingly and locks in a position such that transducer connector 402 is substantially prevented from retracting from transducer interface 302. As shown in FIG. 5, engagement hook 310 is positioned within female engagement feature 312 facilitating the locking of transducer connector 402 to transducer interface 302 when the two are engaged. Engagement hook 310 further facilitates the unlocking of transducer connector 402 from transducer interface 302 responsive to controller 350 providing a release command to actuator 502. In other embodiments, lock engagement mechanism 500 locks transducer connector 402 to to transducer interface 302 in a different manner (e.g., magnetically, via a clamping device, etc.).

Referring to FIGS. 5-6, lock engagement mechanism 500 is configured in a gear arrangement, shown as gear arrangement 600, according to one embodiment. Gear arrangement 600 is configured to couple output shaft 504 to connector shaft 506 to facilitate the transfer of the output from actuator 502 to engagement hook 3 10. As shown in FIGS. 5-6, gear arrangement 600 includes a first gear, shown as gear 602, and a second gear, shown as gear 604. Gear 602 is coupled to an end of output shaft 504 (e.g., the end opposite actuator 502, etc.) and gear 604 is coupled to an end of connector shaft 506 (e.g., the end opposite engagement hook 3 10, etc.). Gear 602 and gear 604 are positioned to interface such that the output of actuator 502 is transferred from gear 602 to gear 604. In one embodiment, gear arrangement 600 is structured to receive a linear output from actuator 502. For example gear arrangement 600 may be structured as a rack and pinon arrangement, among other possibilities, and actuator 502 may be a solenoid, a linear motor, or another type of linear actuator. In another embodiment, gear arrangement 600 is structured to receive a rotational output from actuator 502. For example, gear arrangement 600 may be structured as a spur gear arrangement, a worm gear arrangement, or a helical gear arrangement, among other possibilities, and actuator 502 may be a rotational motor or another type of rotational actuator.

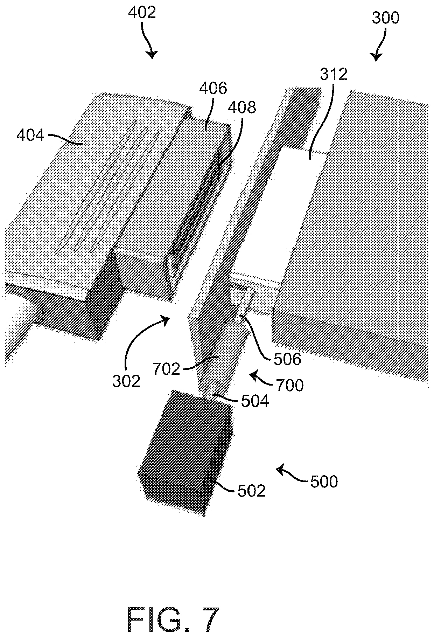

Referring now to FIG. 7, lock engagement mechanism 500 is configured in a linear arrangement, shown as direct connection arrangement 700, according to another embodiment. Direct connection arrangement 700 is configured to couple output shaft 504 to connector shaft 506 to facilitate the transfer of the output from actuator 502 to engagement hook 310. As shown shown in FIG. 7, direct connection arrangement 700 includes a coupling mechanism, shown as coupling 702. Coupling 702 is positioned such that a first end of coupling 702 receives output shaft 504 and an opposing second end receives connector shaft 506, thereby coupling output shaft 504 to connector shaft 506 in a linear arrangement. In one embodiment, direct connection arrangement 700 is structured to receive a linear output from actuator 502. For example, coupling 702 may be structured to receive a linear output from actuator 502 (e.g., a solenoid, a linear motor, etc.) such that the linear output is transferred from actuator 502 to engagement hook 310. In another embodiment, direct connection arrangement 700 is structured to receive a rotational output from actuator 502. For example, coupling 702 may be structured to receive a rotational output from actuator 502 (e.g., a rotational motor, etc.) such that the rotational output is transferred from actuator 502 to engagement hook 310.

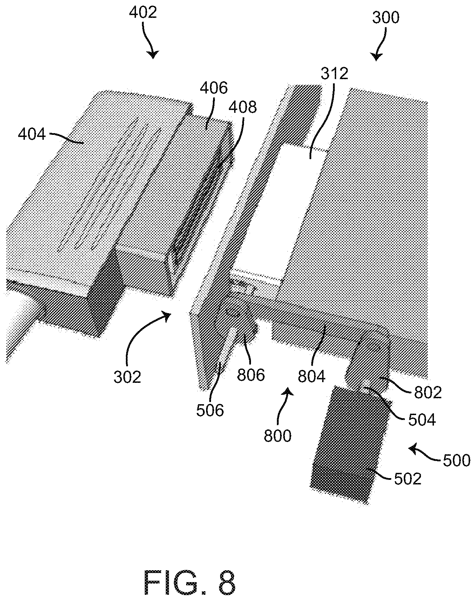

Referring now to FIG. 8, lock engagement mechanism 500 is configured in a linkage arrangement, shown as linkage mechanism arrangement 800, according to yet another embodiment. Linkage mechanism arrangement 800 is configured to couple output shaft 504 to connector shaft 506 to facilitate the transfer of the output from actuator 502 to engagement hook 310. According to the exemplary embodiment shown in FIG. 8, linkage mechanism arrangement 800 is a 3-bar linkage system and includes a first linkage, shown as crank 802, a second linkage, shown as coupler 804, and a third linkage, shown as lever 806. In other embodiments, linkage mechanism arrangement 800 includes a different number of bars/linkages (e.g., a 4-bar linkage system, etc.). As shown in FIG. 8, crank 802 is positioned to rotate about a longitudinal axis defined by output shaft 504 and lever 806 is positioned to rotate about a longitudinal axis defined by connector shaft 506. Coupler 804 is positioned to rotatably couple crank 802 to lever 806 such that a rotation of output shaft 504 is transferred to connector shaft 506, facilitating the transfer of the output from actuator 502 to engagement hook 310 shown in FIG. 5.

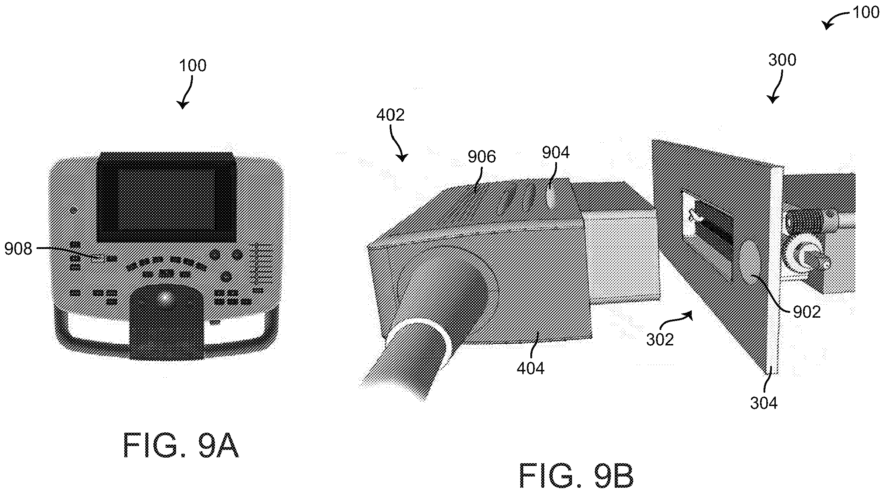

According to the exemplary embodiment shown in FIGS. 9A-9B, ultrasound system 100 and transducer connector 402 may include various release mechanisms (e.g., release mechanism 900, etc.). The release mechanisms are configured to allow a user of ultrasound system 1 00 to release (e.g., unlock, disengage, decouple, etc.) transducer connector 402 from transducer interface 302. As shown in FIG. 9A, a user interface of ultrasound system 100 includes a first button, shown as interface release button 908, configured to provide a release command responsive to being pressed by a user of ultrasound system 100, according to one embodiment. In other embodiments, the user interface may provide the release command responsive to at least one of a touch input to a touch screen, a keyboard input, a voice command, a mouse click, and a mouse motions.

As shown in FIG. 9B, ultrasound system 100 includes a second button, shown as housing release button 902, disposed on housing wall 304, according to another embodiment. In another embodiment, housing release button 902 is configured as a touch sensor. In some embodiments, transducer connector 402 includes a third button, shown a connector release button 904, positioned on connector housing 404. In an alternate embodiment, transducer connector 402 includes a touch sensor, shown as connector touch sensor 906. By way of example, a user may provide an input (e.g., touch, press, etc.) one of housing release button 902, connector release button 904, connector touch sensor 906, and interface release button 908 to disengage transducer connector 402 from transducer interface 302 when he/she wishes to disconnect transducer 400 from ultrasound system 100. By providing an input to one of the aforementioned release mechanisms (e.g., housing release button 902, connector release button 904, connector touch sensor 906, interface release button 908, etc.), a release command is sent by controller 350 to actuator 502 of lock engagement mechanism 500 (see, e.g. controller 350 shown in FIG. 4, actuator 502 and lock engagement mechanism 500 shown in FIGS. 5-8), such that engagement hook 310 is disengaged from transducer connector 402, facilitating the removal of transducer connector 402 from locking system 300. It should be noted that the above mentioned locations of the release mechanisms are exemplary. Therefore, the release mechanisms may be positioned in any suitable location on ultrasound system 100 and/or transducer 400.

In some embodiments, the ultrasound system 100 includes a manual override release mechanism for unlocking the transducer connector 402 from the locking system 300. For example, the manual override release mechanism may be configured to disengage the transducer connector 402 from the transducer interface 302 when there is no power provided to the ultrasound system 100, and/or when there is insufficient power provided to the ultrasound system 100 for using the release mechanisms discussed with references to FIGS.9A-9B. In some embodiments, the manual override release mechanism includes a multi-stage release mechanism, such that multiple actuations of the release mechanism/components thereof are required in order to perform a manual release, thus limiting and/or preventing an accidental manual override.

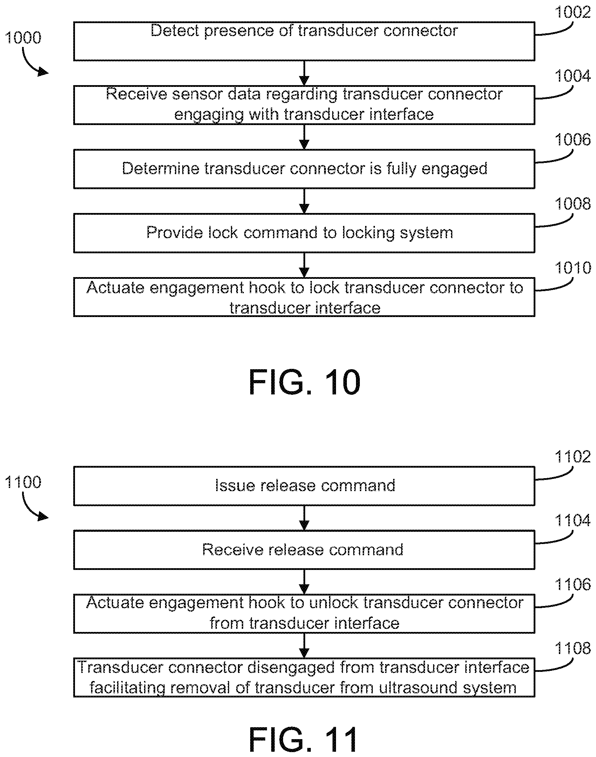

Referring now to FIG. 10, a method 1000 for locking an ultrasound transducer to an ultrasound system is shown according to an example embodiment. Method 1000 corresponds with a controller interpreting sensor data to automatically lock a transducer connector to a transducer interface. In one example embodiment, method 1000 may be implemented with controller 350 of FIG. 4 and locking system 300 of FIGS. 5-8. Accordingly, method 1000 may be described in regard to FIGS. 4-8.

At step 1002, a sensor (e.g., sensor 320, etc.) detects the presence of a transducer connector (e.g., transducer connector 402, etc.). In one embodiment, the sensor is or includes a proximity sensor that is configured to detect the presence of the transducer (e.g., wirelessly, etc.) in the proximity of a transducer interface (e.g., transducer interface 302, etc.). In another embodiment, the sensor is or includes a touch sensor that is configured to detect physical contact of the transducer connector at the transducer interface. At step 1004, controller 350 receives sensor data from the sensor regarding the transducer connector engaging with the transducer interface. For example, a user of an ultrasound system may try to plug a transducer into a transducer interface of the ultrasound system. At step 1006, controller 350 determines whether the transducer connector is fully engaged with the transducer interface (e.g., the components of the interface and the connector are interfacing, connector is fully inserted into the interface, etc.). At step 1008, controller 350 provides a lock command to a locking system (e.g., locking system 300, etc.) responsive to the transducer connector fully engaging the transducer interface. At step 1010, the locking system actuates an engagement hook (e.g., engagement hook 310, etc.) to lock the transducer connector to the transducer interface. The locking system may include a lock engagement mechanism (e.g., lock engagement mechanism 500, etc.) driven by an actuator (e.g., actuator 502, etc.). The lock engagement mechanism may be configured in a gear arrangement, a direct connection arrangement, or a linkage mechanism arrangement.

Referring now to FIG. 11, a method 11 00 for unlocking an ultrasound transducer from an ultrasound system is shown according to an example embodiment. Method 1100 corresponds with controller 350 receiving a release command to unlock a transducer connector from a transducer interface. In one example embodiment, method 1100 may be implemented with controller 350 of FIG. 4, locking system 300 of FIGS. 5-8, and release mechanisms 900 of FIG. 9. Accordingly, method 1100 may be described in regard to FIGS. 4-9.

At step 1102, a user issues a release command via a release mechanism (e.g., release mechanism 900, housing release button 902, connector release button 904, connector touch sensor 906, interface release button 908, etc.). As described above, the release command may be be issued via a mouse click, a button, a touch screen, etc. At step 1104, controller 350 receives the release command. At step 1106, controller 350 provides the release command to a locking system (e.g., locking system 300, etc.) to actuate an engagement hook (e.g., engagement hook 310, etc.) to unlock the transducer connector from a transducer interface. The release command may be communicated to the locking system via a direct mechanical manipulation, a wireless or wired network whereby a processor receives the command and notifies the locking system to release the lock. By way of example, a user may press a button (e.g., housing release button 902, 902, etc.) adjacent to the transducer interface where the button (e.g., mechanically, electronically, etc.) triggers locking system to retract the engagement hook. By way of another example, a user may press a button (e.g., connector release button 904, etc.) or touch a touch sensor (e.g., connector touch sensor 906, etc.) on the transducer connector such that a release command is sent via a wireless network and received by a processor which in turn notifies the locking system to disengage the engagement hook. By way of yet another example, a user may issue a release command through a user interface of the ultrasound system such that the release command is received by a processor through a wired network. The processor then notifies the locking system to disengage the engagement hook retract, facilitating the decoupling of the transducer connector form the ultrasound system. At step 1108, the transducer connector is disengaged from the transducer interface facilitating the removal of the transducer from the ultrasound system.

It should be noted that the term "example" or "exemplary" as used herein to describe various embodiments is intended to indicate that such embodiments are possible examples, representations, and/or illustrations of possible embodiments (and such term is not intended to connote that such embodiments are necessarily extraordinary or superlative examples).

The terms "coupled," "connected," and the like, as used herein, mean the joining of two members directly or indirectly to one another. Such joining may be stationary (e.g., permanent) or moveable (e.g., removable, releasable, etc.). Such joining may be achieved with the two members or the two members and any additional intermediate members being integrally formed as a single unitary body with one another or with the two members or the two members and any additional intermediate members being attached to one another.

References herein to the positions of elements (e.g., "top," "bottom," "above," "below," etc.) are merely used to describe the orientation of various elements in the figures. It should be noted that the orientation of various elements may differ according to other exemplary embodiments, and that such variations are intended to be encompassed by the present disclosure.

The present disclosure contemplates methods, systems, and program products on any machine-readable media for accomplishing various operations. The embodiments of the present present disclosure may be implemented using existing computer processors, or by a special purpose computer processor for an appropriate system, incorporated for this or another purpose, or by a hardwired system. Embodiments within the scope of the present disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise comprise RAM, ROM, EPROM, EEPROM, CD-ROM or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general purpose computer, special purpose computer, or special purpose processing machines to perform a certain function or group of functions.

It is important to note that the construction and arrangement of the elements of the systems and methods as shown in the exemplary embodiments are illustrative only. Although only a few embodiments of the present disclosure have been described in detail, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter recited. For example, elements shown as integrally formed may be constructed of multiple parts or elements. It should be noted that the elements and/or assemblies of the components described herein may be constructed from any of a wide variety of materials that provide sufficient strength or durability, in any of a wide variety of colors, textures, and combinations.

Any comparative embodiments or embodiments that are not covered by the appended claims are described for facilitating understanding of the present application.

## Claims

1. An apparatus for locking a transducer connector (402) to a transducer interface (302) of an ultrasound system (100), comprising:
a sensor (320) configured to acquire data regarding a position of the transducer connector (402) relative to the transducer interface (302);
a locking system (300) configured to facilitate locking of the transducer connector (402) to the transducer interface (302); and
a processing circuit (163) communicably coupled to the sensor (320) and the locking system (300), the processing circuit (163) configured to:
interpret the data acquired by the sensor (320); and
provide a lock command to the locking system (300) to lock the transducer connector (402) to the transducer interface (302) based on the data indicating the transducer connector (402) is engaged with the transducer interface (302);
**characterized in that**, the locking system (300) comprises an indicator configured to:
flash a designated color when it is determined that the transducer connector (402) is not in mechanical and software compatibility with the ultrasound system (100) / the locking system (300);
flash a different designated color when it is determined that the transducer connector (402) is in mechanical and software compatibility with the ultrasound system (100) / the locking system (300).

2. The apparatus of claim 1, wherein the sensor (320) includes at least one of:
a proximity senor configured to detect a presence of the transducer connector (402) in proximity of the transducer interface (302); and
a touch sensor configured to detect physical contact from the transducer connector (402).

3. The apparatus of claim 1, wherein the locking system (300) includes an actuator (502) and a lock engagement mechanism (500), wherein the actuator (502) is configured to provide an output that actuates the lock engagement mechanism (500) such that the lock engagement mechanism (500) engages or disengages the transducer connector (402).

4. The apparatus of claim 3, wherein the output actuates the lock engagement mechanism (500) to implement one of the following:
the lock engagement mechanism (500) engages the transducer connector (402) responsive to the lock command; and
the lock engagement mechanism (500) disengages the transducer connector (402) responsive to a release command.

5. The apparatus of claim 3, wherein the lock engagement mechanism (500) is configured as at least one of a gear arrangement (600), a direct connection arrangement (700), or a linkage mechanism arrangement (800).

6. The apparatus of claim 3, wherein the lock engagement mechanism (500) includes an engagement hook (310) configured to directly couple the transducer connector (402) responsive to the lock command.

7. The apparatus of claim 3, further comprising a release mechanism (900) configured to provide a release command to the processing circuit (163).

8. A method for locking a transducer connector (402) to a transducer interface (302) of an ultrasound system (100), comprising:
acquiring, by a sensor (320), data regarding a position of the transducer connector (402) relative to the transducer interface (302);
interpreting, by a processing circuit (163), the data;
providing, by the processing circuit (163), a lock command to a locking system (300) to lock the transducer connector to the transducer interface (302) based on the data indicating the transducer connector is engaged with the transducer interface (302);
**characterized in that**, the method further comprises:
flashing, by an indicator, a designated color when it is determined that the transducer connector (402) is not in mechanical and software compatibility with the ultrasound system (100) / the locking system (300);
flashing, by the indicator, a different designated color when it is determined that the transducer connector (402) is in mechanical and software compatibility with the ultrasound system (100) / the locking system (300).

9. The method of claim 8, wherein the sensor (320) includes at least one of:
a proximity senor configured to detect a presence of the transducer connector (402) in proximity of the transducer interface (302); and
a touch sensor configured to detect physical contact from the transducer connector (402).

10. The method of claim 8, further comprising actuating, by an actuator (502) of the locking system (300), a lock engagement mechanism (500) to implement one of the following:
the lock engagement mechanism (500) engages the transducer connector (402) responsive to receiving the lock command, and
the lock engagement mechanism (500) disengages from the transducer connector (402) responsive to receiving a release command.

11. The method of claim 10, wherein the lock engagement mechanism (500) is configured as at least one of a gear arrangement (600), a direct connection arrangement (700), or a linkage mechanism arrangement (800).

12. The method of claim 10, wherein the lock engagement mechanism (500) includes an engagement hook (310) configured to directly couple the transducer connector (402) responsive to the lock command.

13. The method of claim 8, further comprising:
receiving, by a release mechanism (900), an input from a user; and
providing, by the processing circuit (163), a release command to the locking system (300) responsive the input.

## Patentansprüche

1. Vorrichtung zum Verriegeln eines Wandlersteckers (402) mit einem Wandlerinterface (302) eines Ultraschallsystems (100), mit:
einem Sensor (320), der dazu ausgebildet ist, Daten bezüglich einer Position des Wandlersteckers (402) in Bezug auf das Wandlerinterface (302) zu erfassen;
einem Verriegelungssystem (300), das dazu ausgebildet ist, das Verriegeln des Wandlersteckers (402) mit dem Wandlerinterface (302) zu vereinfachen; und
einer Verarbeitungsschaltung (163), die kommunikationsfähig mit dem Sensor (320) und dem Verriegelungssystem (300) gekoppelt ist, wobei die Verarbeitungsschaltung (163) dazu ausgebildet ist:
die von dem Sensor (320) erfassten Daten zu interpretieren; und
auf der Basis, dass die Daten das Zusammengreifen des Wandlersteckers (402) mit dem Wandlerinterface (302) angeben, einen Verriegelungsbefehl an das Verriegelungssystem (300) auszugeben, um den Wandlerstecker (402) mit dem Wandlerinterface (302) zu verriegeln,
**dadurch gekennzeichnet, dass** das Verriegelungssystem (300) eine Anzeige aufweist, die dazu ausgebildet ist:
eine vorbestimmte Farbe aufleuchten zu lassen, wenn festgestellt wird, dass der Wandlerstecker (402) keine mechanische und softwaremäßige Kompatibilität mit dem Ultraschallsystem (100)/dem Verriegelungssystem (300) aufweist;
eine andere vorbestimmte Farbe aufleuchten zu lassen, wenn festgestellt wird, dass der Wandlerstecker (402) eine mechanische und softwaremäßige Kompatibilität mit dem Ultraschallsystem (100)/dem Verriegelungssystem (300) aufweist.

2. Vorrichtung nach Anspruch 1, bei welcher der Sensor (320) aufweist:
einen Näherungssensor, der dazu ausgebildet ist, das Vorhandensein des Wandlersteckers (402) in der Nähe des Wandlerinterface (302) zu detektieren; und/oder
einen Touch-Sensor, der dazu ausgebildet ist, physischen Kontakt seitens des Wandlersteckers (402) zu detektieren.

3. Vorrichtung nach Anspruch 1, bei welcher das Verriegelungssystem (300) einen Aktuator (502) und einen Verriegelungseingriffsmechanismus (500) aufweist, wobei der Aktuator (502) dazu ausgebildet ist, eine Ausgabe zu liefern, welche den Verriegelungseingriffsmechanismus (500) derart betätigt, dass der Verriegelungseingriffsmechanismus (500) an dem Wandlerstecker (402) angreift oder diesen freigibt.

4. Vorrichtung nach Anspruch 3, bei welcher die Leistung den Verriegelungseingriffsmechanismus (500) betätigt, um eine der folgenden Aktionen zu implementieren:
der Verriegelungseingriffsmechanismus (500) greift an dem Wandlerstecker (402) in Reaktion auf den Verriegelungsbefehl an; und
der Verriegelungseingriffsmechanismus (500) gibt den Wandlerstecker (402) in Reaktion auf einen Entriegelungsbefehl frei.

5. Vorrichtung nach Anspruch 3, bei welcher der Verriegelungseingriffsmechanismus (500) als eine Getriebeanordnung (600) und/oder eine Direktverbindungsanordnung (700) oder eine Kopplungsmechanismusanordnung (800) ausgebildet ist.

6. Vorrichtung nach Anspruch 3, bei welcher der Verriegelungseingriffsmechanismus (500) einen Eingreifhaken (310) aufweist, der dazu ausgebildet ist, den Wandlerstecker (402) in Reaktion auf den Verriegelungsbefehl direkt zu verbinden.

7. Vorrichtung nach Anspruch 3, ferner mit einem Entriegelungsmechanismus (900), der dazu ausgebildet ist, einen Entriegelungsbefehl an die Verarbeitungsschaltung (163) auszugeben.

8. Verfahren zum Verriegeln eines Wandlersteckers (402) mit einem Wandlerinterface (302) eines Ultraschallsystems (100), mit den folgenden Schritten:
Erfassen, durch einen Sensor (320), von Daten bezüglich einer Position des Wandlersteckers (402) in Bezug auf das Wandlerinterface (302);
Interpretieren der Daten durch eine Verarbeitungsschaltung (163);
auf der Basis, dass die Daten das Zusammengreifen des Wandlersteckers mit dem Wandlerinterface (302) angeben, Ausgeben eines Verriegelungsbefehls an ein Verriegelungssystem (300) durch die Verarbeitungsschaltung (163), um den Wandlerstecker mit dem Wandlerinterface (302) zu verriegeln;
**dadurch gekennzeichnet, dass** das Verfahren ferner die folgenden Schritte aufweist:
Aufleuchtenlassen einer vorbestimmten Farbe durch eine Anzeige, wenn festgestellt wird, dass der Wandlerstecker (402) keine mechanische und softwaremäßige Kompatibilität mit dem Ultraschallsystem (100)/dem Verriegelungssystem (300) aufweist;
Aufleuchtenlassen einer anderen vorbestimmten Farbe durch die Anzeige, wenn festgestellt wird, dass der Wandlerstecker (402) eine mechanische und softwaremäßige Kompatibilität mit dem Ultraschallsystem (100)/dem Verriegelungssystem (300) aufweist.

9. Verfahren nach Anspruch 8, bei welchem der Sensor (320) aufweist:
einen Näherungssensor, der dazu ausgebildet ist, das Vorhandensein des Wandlersteckers (402) in der Nähe des Wandlerinterface (302) zu detektieren; und/oder
einen Touch-Sensor, der dazu ausgebildet ist, physischen Kontakt seitens des Wandlersteckers (402) zu detektieren.

10. Verfahren nach Anspruch 8, ferner mit dem Betätigen eines Verriegelungseingriffsmechanismus (500) durch einen Aktuator (502) des Verriegelungssystems (302), um eine der folgenden Aktionen zu implementieren:
der Verriegelungseingriffsmechanismus (500) greift an dem Wandlerstecker (402) in Reaktion auf den Verriegelungsbefehl an; und
der Verriegelungseingriffsmechanismus (500) gibt den Wandlerstecker (402) in Reaktion auf einen Entriegelungsbefehl frei.

11. Verfahren nach Anspruch 10, bei welchem der Verriegelungseingriffsmechanismus (500) als eine Getriebeanordnung (600) und/oder eine Direktverbindungsanordnung (700) oder eine Kopplungsmechanismusanordnung (800) ausgebildet ist.

12. Verfahren nach Anspruch 10, bei welchem der Verriegelungseingriffsmechanismus (500) einen Eingreifhaken (310) aufweist, der dazu ausgebildet ist, den Wandlerstecker (402) in Reaktion auf den Verriegelungsbefehl direkt zu verbinden.

13. Verfahren nach Anspruch 8, ferner mit den Schritten:
Empfangen einer Eingabe von einem Benutzer durch den Entriegelungsmechanismus (900); und
Ausgeben eines Entriegelungsbefehls durch die Verarbeitungsschaltung (163) an das Verriegelungssystem (300) in Reaktion auf die Eingabe.

## Revendications

1. Appareil de verrouillage d'un connecteur de transducteur (402) à une interface de transducteur (302) d'un système à ultrasons (100), comprenant :
un capteur (320) configuré pour acquérir des données concernant une position du connecteur de transducteur (402) par rapport à l'interface de transducteur (302) ;
un système de verrouillage (300) configuré pour faciliter le verrouillage du connecteur de transducteur (402) à l'interface de transducteur (302) ; et
un circuit de traitement (163) couplé de manière communicative au capteur (320) et au système de verrouillage (300), le circuit de traitement (163) étant configuré pour :
interpréter les données acquises par le capteur (320) ; et
fournir une commande de verrouillage au système de verrouillage (300) pour verrouiller le connecteur de transducteur (402) à l'interface de transducteur (302) sur la base des données indiquant que le connecteur de transducteur (402) est mis en prise avec l'interface de transducteur (302) ;
**caractérisé en ce que** le système de verrouillage (300) comprend un indicateur configuré pour :
faire clignoter une couleur désignée lorsqu'il est déterminé que le connecteur de transducteur (402) n'est pas en compatibilité mécanique et logicielle avec le système à ultrasons (100) / le système de verrouillage (300) ;
faire clignoter une couleur désignée différente lorsqu'il est déterminé que le connecteur de transducteur (402) est en compatibilité mécanique et logicielle avec le système à ultrasons (100) / le système de verrouillage (300).

2. Appareil selon la revendication 1, dans lequel le capteur (320) comporte :
un capteur de proximité configuré pour détecter une présence du connecteur de transducteur (402) à proximité de l'interface de transducteur (302) ; et/ou
un capteur tactile configuré pour détecter un contact physique depuis le connecteur de transducteur (402).

3. Appareil selon la revendication 1, dans lequel le système de verrouillage (300) comporte un actionneur (502) et un mécanisme de mise en prise de verrouillage (500), l'actionneur (502) étant conçu pour fournir une sortie qui actionne le mécanisme de mise en prise de verrouillage (500) de telle sorte que le mécanisme de mise en prise de verrouillage (500) met en prise ou désolidarise le connecteur de transducteur (402).

4. Appareil selon la revendication 3, dans lequel la sortie actionne le mécanisme de mise en prise de verrouillage (500) pour mettre en œuvre l'un des éléments suivants :
le mécanisme de mise en prise de verrouillage (500) met en prise le connecteur de transducteur (402) en réponse à la commande de verrouillage ; et
le mécanisme de mise en prise de verrouillage (500) désolidarise le connecteur de transducteur (402) en réponse à une commande de libération.

5. Appareil selon la revendication 3, dans lequel le mécanisme de mise en prise de verrouillage (500) est conçu comme un agencement d'engrenage (600), et/ou un agencement de liaison directe (700) et/ou un agencement de mécanisme de liaison (800).

6. Appareil selon la revendication 3, dans lequel le mécanisme de mise en prise de verrouillage (500) comporte un crochet de mise en prise (310) conçu pour accoupler directement le connecteur de transducteur (402) en réponse à la commande de verrouillage.

7. Appareil selon la revendication 3, comprenant en outre un mécanisme de libération (900) conçu pour fournir une commande de libération au circuit de traitement (163).

8. Procédé de verrouillage d'un connecteur de transducteur (402) à une interface de transducteur (302) d'un système à ultrasons (100), comprenant :
l'acquisition, par un capteur (320), de données concernant une position du connecteur de transducteur (402) par rapport à l'interface de transducteur (302) ;
l'interprétation, par un circuit de traitement (163), des données ;
la fourniture, par le circuit de traitement (163), d'une commande de verrouillage à un système de verrouillage (300) pour verrouiller le connecteur de transducteur à l'interface de transducteur (302) sur la base des données indiquant que le connecteur de transducteur est mis en prise avec l'interface de transducteur (302) ;
**caractérisé en ce que** le procédé comprend en outre :
le clignotement, par un indicateur, d'une couleur désignée lorsqu'il est déterminé que le connecteur de transducteur (402) n'est pas en compatibilité mécanique et logicielle avec le système à ultrasons (100) / le système de verrouillage (300) ;
le clignotement, par l'indicateur, d'une couleur désignée différente lorsqu'il est déterminé que le connecteur de transducteur (402) est en compatibilité mécanique et logicielle avec le système à ultrasons (100) / le système de verrouillage (300).

9. Procédé selon la revendication 8, dans lequel le capteur (320) comporte :
un capteur de proximité configuré pour détecter une présence du connecteur de transducteur (402) à proximité de l'interface de transducteur (302) ; et/ou
un capteur tactile configuré pour détecter un contact physique depuis le connecteur de transducteur (402).

10. Procédé selon la revendication 8, comprenant en outre l'actionnement, par un actionneur (502) du système de verrouillage (300), d'un mécanisme de mise en prise de verrouillage (500) pour mettre en œuvre l'un des éléments suivants :
le mécanisme de mise en prise de verrouillage (500) met en prise le connecteur de transducteur (402) en réponse à la réception de la commande de verrouillage, et
le mécanisme de mise en prise de verrouillage (500) se désolidarise du connecteur de transducteur (402) en réponse à la réception d'une commande de libération.

11. Procédé selon la revendication 10, dans lequel le mécanisme de mise en prise de verrouillage (500) est conçu comme un agencement d'engrenage (600), et/ou un agencement de liaison directe (700) et/ou un agencement de mécanisme de liaison (800).

12. Procédé selon la revendication 10, dans lequel le mécanisme de mise en prise de verrouillage (500) comporte un crochet de mise en prise (310) conçu pour accoupler directement le connecteur de transducteur (402) en réponse à la commande de verrouillage.

13. Procédé selon la revendication 8, comprenant en outre :
la réception, par un mécanisme de libération (900), d'une entrée provenant d'un utilisateur ; et
la fourniture, par le circuit de traitement (163), d'une commande de libération au système de verrouillage (300) en réponse à l'entrée.
